# EUROPEAN PATENT SPECIFICATION

(11) **EP 3 750 165 B1**
(45) Date of publication and mention of the grant of the patent: **24.04.2024**
(21) Application number: 19700916.0
(22) Date of filing: 16.01.2019
(51) Int. Cl.: G16H 40/60, G16H 40/20

(54) **METHOD FOR REGISTERING A USER IN A MEDICAL SOFTWARE APPLICATION**
VERFAHREN ZUR REGISTRIERUNG EINES BENUTZERS EINER MEDIZINISCHEN SOFTWAREANWENDUNG
PROCÉDÉ POUR ENREGISTRER UN UTILISATEUR DANS UNE APPLICATION DE LOGICIEL MÉDICAL

(30) Priority: 09.02.2018 EP 18305137
(43) Date of publication of application: 16.12.2020
(73) Proprietor: Fresenius Vial SAS, 38590 Brézins (FR)
(72) Inventor: LAMBERT, William, 38300 Bourgoin Jallieu (FR); BOYER, Pierrick, 38470 Vinay (FR); DUPARCHY, Bertrand, 38430 Moirans (FR)
(74) Representative: Fresenius Kabi Deutschland GmbH
(86) International application number: PCT/EP2019/051026
(87) International publication number: WO 2019/154600

(56) References cited:
- US-A1- 2014 189 346
- US-A1- 2015 215 889
- "M-Audio Account", , 31 December 2017 (2017-12-31), XP93046350, Retrieved from the Internet: URL:https://web.archive.org/web/2017123120 0842/http://www.m-audio.com:80/account/sof tware-instructions [retrieved on 2023-05-11]

## Description

The invention relates to a method for registering a user in a medical software application, to a computing device configured to execute such a method, and to a software program product implementing such a method.

A medical software application may in particular relate to the operation and control of medical devices, such as medical infusion devices for administering a medical fluid, e.g., a medication or a nutritional solution for the enteral feeding, to a patient. A medical software application of this kind may in particular relate to drug libraries configured to control the programming of infusion devices, the software application for example enabling a user to create, edit or share drug libraries within a hospital environment or between multiple different, physically separated hospital environments, such as different hospitals.

Typically, medical devices for administering drugs to a patient, such as infusion pumps, are installed at various locations in a healthcare environment, for example in a hospital facility. Such medical devices may for example be located in different rooms of wards (care units) of a hospital or in operating rooms. Nowadays, such medical devices are connected to a local network for communicating with a hospital management system hosted on a server located in the healthcare environment. For example, a group of infusion pumps may be installed on a rack serving as a communication link to the local network such that via the rack the infusion pumps are connected to the local network and are operative to communicate with a hospital management system on a server within the healthcare environment, for example within a hospital, via the local network, for example a local area network (LAN) or a wireless local area network (WLAN).

To control the operation of medical devices of this kind for administering drugs to a patient, so called drug libraries are installed on such medical devices, a drug library comprising drug library data characterizing a drug, its ingredients, rules for compatibility and rules for administration or the like. A drug library may for example comprise a list of drugs in which each drug is associated with parameters defining for example boundary values for administration by means of an infusion device. Such boundary values may for example relate to a minimum and maximum dosage for administering a particular drug, a minimum and maximum rate for administering a drug, a minimum and maximum time of administration and the like. In addition, such boundary values may be dependent on the age, weight and gender of a patient and, hence, may be patient-specific.

By using such drug libraries the operation of a medical device such as an infusion pump for administering a particular drug to a patient is controlled in that the medical device may be operated by a nurse only within the boundaries posed by the drug library. For this, for administering a drug to a patient, the nurse identifies the drug to the medical device, upon which the medical device automatically loads the respective rules and boundary values from a drug library installed on the medical device.

Presently, such drug libraries are locally installed within a healthcare environment, for example within a hospital. Such drug libraries are for example installed as software on a personal computer (PC) or a server within a hospital, from which the drug library may be distributed to medical devices located in different wards of a hospital in order to be installed on such medical devices.

WO 2005/036447 A2 discloses a medication management system including a medication management unit associated with a medical device. The medication management unit is set up to compare a medication order information from a first input means to machine readable delivery information from a second input means and to download a medication order to the medical device only if the information from the first input means matches the information from the second input means. The medication management unit also comprises a drug library editor enabling a user to import, export and edit whole drug libraries and individual drug library values to control and customize a drug library according to hospital preferences.

From WO 2010/132617 A2 a computer-implemented method of interacting with a medical device in conjunction with a user device is known. Within the method a certified medical application is received at a user device and is stored in a secure memory segment. A communication link is established from the user device to the medical device in order to execute the certified medical application on the medical device.

US2015215889A1 addresses a registration, verification and notification system for registering a user in a software application.

As computing devices such as a personal computer located within a hospital environment may run in the local environment of a hospital and possibly shall not use a connection to for example the Internet, the problem arises that user accounts for accessing a software application running on such a computing device must be set up locally, without making use of an external network connection. This typically involves the technical issues of identifying the user accessing the software application, and in addition of checking the validity of a license based on which the user wishes to use the software application.

In today's software applications used in particular in a corporate environment, it is typical that a privileged user (also denoted as administrator) has the right to enter user credentials and license keys into a software application in order to set up a user account allowing a user to access a software application and to make use of the software application according to privileges associated with the user account. In particular in a medical environment in which software applications are used offline, i.e., without a network connection to a public communication network such as the Internet, there however is a desire to ease the creation of user accounts and to improve the management of licenses to simplify workflows and to be able to have an improved license control.

It is an object of the instant invention to provide a method, a computing device and a software program product which allow for an easy management of user accounts and licenses within a medical software application.

This object is achieved by means of a method comprising the features of claim 1. Accordingly, the method comprises:
- providing a registration code to a user, the registration code containing information relating to a license key and information relating to at least one user credential associated with the user,
- entering, by the user, the registration code and at least one user credential associated with the user into the software application,
- processing, by the software application, the registration code and the at least one user credential entered by the user, and
- creating a user account for the user in the software application based on the information relating to the at least one user credential associated with the user contained in the registration code.

By making use of the method, the set up and creation of user accounts becomes possible without involving a privileged user (administrator). Rather, the creation of a user account within a software application can take place by providing a registration code to a user, the registration code containing information relating to the license key and information relating to at least one user credential associated with the user. Such registration code may for example be provided to the user from the software supplier of the medical software application, wherein the software supplier may provide the registration code to the user upon request by the user and upon obtaining user credentials, such as a username and a password, from the user.

Upon obtaining the registration code, the user enters the registration code into the software application, for example via a login screen of the software application. In addition, together with entering the registration code the user is required to enter its user credentials into the software application, such as the username and password, as the user has provided them previously to the software supplier. Hence, both the registration code and the user credentials are entered into the software application by the user.

Once the user has entered the registration code and the user credentials into the software application, the software application processes the registration code and the user credentials and, if the processing is successful, creates the user account. In particular, the software application may process the registration code in order to derive the user credentials from the registration code. The processing may then involve comparing the user credentials as derived from the registration code and the user credentials which have been entered by the user into the software application. If the user credentials derived from the registration code and the user credentials entered by the user into the system match, a user account for the user is created in the software application based on the user credentials, for example based on a combination of a username and a password.

Hence, the creation of a user account becomes possible based on the entering of the registration code by the user into the software application. The registration code is obtained from for example the software supplier, such that for example within a hospital environment no privileged user (administrator) needs to be involved in order to create the user account.

In one embodiment, the registration code is encrypted making use of a known encryption technology, such as an RSA encryption technology. The registration code hence is provided to the user not in a clear-text form, but in an encrypted form, such that no third-party may derive any information from the registration code in an obvious manner. In this regard the software application is configured to decrypt the registration code when it is entered by the user into the software application, such that the software application may derive knowledge about the license key and the user credentials contained in the registration code, enabling the software application to check the license validity and to verify the user credentials derived from the registration code versus the user credentials entered by the user into the system.

Because of the processing of the registration code (which advantageously is encrypted when it is supplied to the user) in order to derive information relating to the license key and information relating to the user credentials from the registration code, an improved licensing control becomes possible. In particular, even if the registration code becomes known to a third-party, for example during the process of transferring the registration code from the software supplier to the user, the third-party is not able to make use of the registration code, because the registration code needs to be entered into the software application by the user together with the user credentials. If the third-party does not have knowledge about the user credentials, the processing of the registration code by the software application will not succeed, because the user credentials contained in the registration code cannot be verified with respect to user credentials entered into the software application.

In one embodiment, the processing by the software application includes at least one of checking the validity of the registration code, verifying the information relating to the license key contained in the registration code, and verifying the information relating to the at least one user credential contained in the registration code with respect to the at least one user credential entered by the user into the software application.

The validity of the registration code may for example be checked without decoding the registration code. In particular, for checking the validity of the registration code the format of the registration code, for example its length and data format, may be checked, and if and only if the format of the registration code matches certain criteria, it is found valid.

The registration code may in particular be a string of characters involving letters and numbers. The registration code may have a predefined length and may comprise predefined portions.

The verifying of the information relating to the license key contained in the registration code may for example include verifying the information relating to the license key with respect to a valid license key stored in the software application. The software application may for example have a list of valid license keys stored. The license key derived from the registration code may hence be checked against the list of valid license keys, and if a match is found, the license key is assumed valid. Alternatively, the software application may be able to verify a license key by matching it to certain criteria, which may involve for example a processing of the license key making use of a predefined algorithm.

The verifying of the information relating to the user credentials contained in the registration code may involve comparing the user credentials as derived from the registration code to the user credentials entered by the user into the software application together with the registration code. For example, the software application may, by decoding the registration code, derive a username and a password from the registration code, the username and password being contained in the registration code as it has been provided by the software supplier to the user. In addition, the user enters its username and password into the software application together with the registration code, such that the software application may check whether the username and the password as entered by the user matches the username and password as derived from the registration code. If and only if the user credentials as entered by the user and as derived from the registration code match, the processing of the registration code and the user credentials yields a successful result, upon which the user account may be created.

In particular, the user account is, in one embodiment, created if and only if the processing of the registration code is successful. In particular, condition for creating the user account may be that all processing steps, for example relating to the checking of the validity of the registration code, the verifying of the license key contained in the registration code and the verifying of the user credentials yields a successful result.

The creating of the user account may involve for example the storing of the user credentials in the software application. For example, the software application may have an internal database in which user account information is stored, the user accounts being identified for example by the user names of different user, wherein each user name is associated with a password enabling the user to access its user account.

It is not necessary that all user credentials are contained in the registration code. For example, the registration code may contain, in one embodiment, only information relating to the username, but not the password. It however is also possible that the registration code contains information relating both to the username and the password.

In one embodiment, the registration code may contain additional information, for example relating to identification information, such as a MAC address, of a computing device on which the user is authorized to execute the software application and/or information related to functions of the software application the user is authorized to access or execute and/or information relating to a validity period of the license key. Such information may be stored in association with the user account upon creating the user account, such that it is defined for the user account from which computing device the user may access the software application, which functions of the software application the user may be authorized to use and/or for what time period the user may be enabled to access the software application.

The object is also achieved by means of a computing device configured to execute the method as described above.

The object is also achieved by a software program product implementing the method as described above.

The communication between for example the software supplier and the user for providing the registration code to the user may take place in different ways, for example via a public communication network by sending an electronic message to the user, or by making use of any other known communication technology, may it be electronic (i.e., via an electronic communication network) or not (e.g., by regular mail).

The idea underlying the invention shall subsequently be described in more detail with reference to the embodiments of the figures. Herein:
- Fig. 1: shows a schematic drawing of a scenario as it can be found in a hospital environment;
- Fig. 2: shows a schematic drawing of a registration code;
- Fig. 3: shows a flow diagram of a method for registering a user in a medical software application; and
- Fig. 4: shows a schematic drawing of a computing device configured to execute a software application.

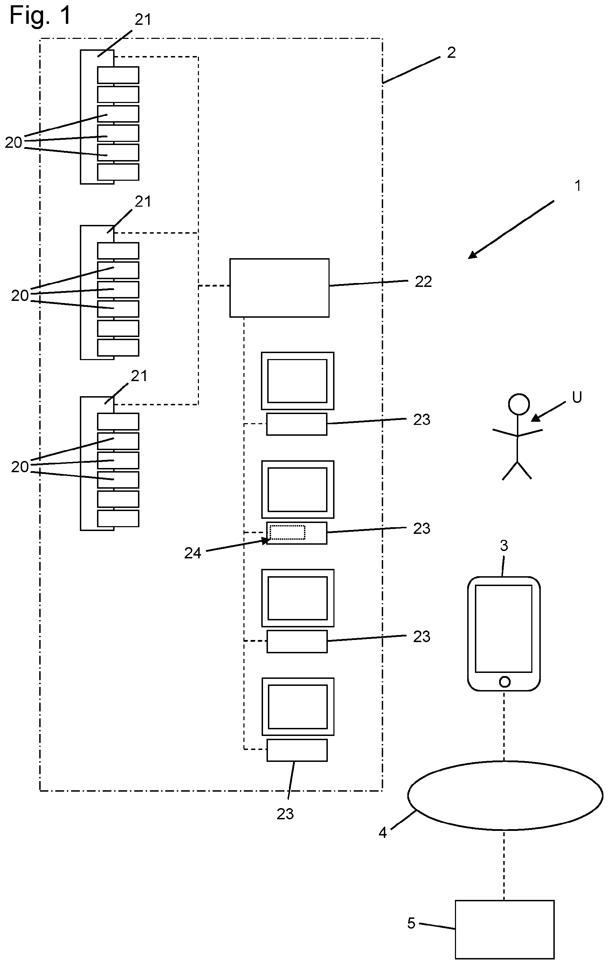

Fig. 1 shows, in a schematic drawing, a setup as it may be found in a hospital environment 2.

A system 1, as it is set up in a hospital environment 2, for example comprises a multiplicity of medical devices 20, for example in the shape of infusion devices, such as peristaltic (volumetric) or syringe infusion pumps, located for example in an organized fashion on organization devices 21 such as racks in different bedrooms, units and wards of the hospital environment 2, for example within a hospital. The organization devices 21 may for example form a vertical stack of medical devices 20 by mechanically holding the medical devices 20 and by in addition providing for a power and communication link for the medical devices 20 by connecting the medical devices 20 to an external power source and by communicatively connecting the medical devices 20 via an internal, local communication network to a central server 22 of the hospital environment 2, the communication network together with the central server 22 for example forming an hospital information management system (HIMS) for managing information and data flow within the hospital environment 2.

Clinic personnel may access the hospital information management system for example using computing devices 23 in the shape of for example personal computers (PCs). For example, a user U may access a computing device 23 to run software applications installed on the computing device 23 for controlling the operation of one or multiple medical devices 20, for entering information into the system or for obtaining information from the system. A software application of this kind may for example be a software application relating to drug libraries as they are used by medical devices 20 such as infusion devices during operation for administering certain drugs to a patient, the software application enabling a user U for example to create new drug libraries, to edit existing drug libraries and to share drug libraries between multiple different medical devices 20 within the hospital environment 2.

In the context of this text a drug library is to be understood as a list of drugs in which each of the drugs is associated with parameters defining operational boundaries for administering the particular drug to a patient. Such parameters may depend on the patient demographics, for example the patient's age, weight or gender. Drug libraries, as they are conventionally known, serve to provide rules to medical devices 20 for administering drugs to a patient. In particular, drug libraries in the context of infusion devices contain a list of drugs in which each drug is associated with parameters that define, characterize and impose boundary values on an infusion device for administering the particular drug to a patient. For example, such boundary values may relate to the dosage, the rate of administration and the time of administration for a drug and may vary for different drugs and also for different types of patients, for example dependent on the age, weight and gender of a patient.

There is a desire to provide for an easy workflow in order to allow a user to access a software application, the workflow advantageously being designed to ease the setup of a user account and to improve license control, in particular preventing an unauthorized third-party to access a software application in an unauthorized manner.

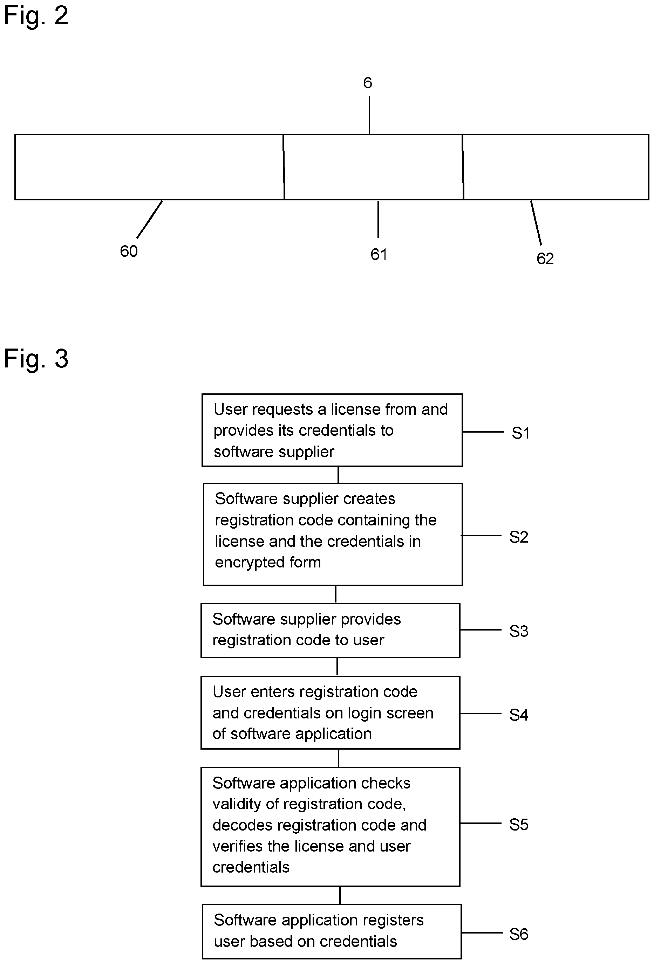

In this context, for registering a user in a medical software application a workflow as illustrated in an embodiment in Fig. 3 is proposed in which a registration code 6 as it is schematically shown in Fig. 2 is provided to a user U from an external communication entity 5 associated for example with the software supplier of the software application to be accessed, the transfer of the registration code 6 for example taking place via a public communication network 4 such as the Internet to a communication device 3, for example a smart device such as a smart phone or a portable computing device such as a laptop or tablet computer, of the user U.

The registration code 6, as illustrated in Fig. 2, comprises different portions relating to information 60 about a license key and to information 61, 62 about user credentials such as a username and a password. The registration code 6 is generated by the software supplier and is transmitted to the user U, in the example illustrated in Fig. 1 to the communication device 3 of the user U, by the software supplier.

For this, the user U may have to request the registration code 6 (step S1 in Fig. 3) and may have to transmit user credentials to the software supplier, such that the software supplier is able to generate the registration code 6 by including information 61, 62 about the user credentials. Following the request, the software supplier generates the registration code 6 (step S2 in Fig. 3) and transmits the registration code 6 containing information 60-62 about the license and the user credentials to the user U (step S3 in Fig. 3).

The registration code 6 may have the shape of a string of characters, comprising letters and numbers, and may have a defined length. The registration code 6 may in particular be generated in an encrypted form making use of a known encryption technology such as an RSA encryption technology. The registration code 6 hence is not transmitted in a clear-text format, but is encrypted, such that a third-party may not derive information from the registration code 6 if the third-party intercepts the transmission of the registration code 6.

Upon obtaining the registration code 6, the user U enters the registration code 6 into the software application by accessing the software application on a computing device 23 (see Fig. 1). The user U may enter the registration code 6 for example via a login screen provided by the software application and is required to enter, together with the registration code 6, its user credentials into the software application, as they have been provided to the software supplier for generating the registration code 6 (step S4 in Fig. 3).

Once the user U has entered the registration code 6 and its user credentials into the software application running on the computing device 23, the software application processes the registration code 6 by checking the validity of the registration code, for example by checking whether the registration code 6 adheres to a predefined, required format. In addition, the software application decrypts the registration code 6 in order to derive the information 60 relating to the license key and the information 61, 62 relating to the user credentials from the registration code 6, such that the software application may verify the license key and may verify the user credentials as derived from the registration code 6 versus the user credentials as the user U has entered them into the system (step S5 in Fig. 3).

If and only if the processing yields a positive result, the software application registers the user U in the software application by creating a user account (step S6 in Fig. 3). In particular, only if the registration code 6 as such is found valid and in addition if the license key matches predefined criteria, and in addition if the user credentials as they have been derived from the registration code 6 match the user credentials as the user U has entered them into the software application, the user account is created, such that the user U is enabled to log into the software application to make use of the software application, for example to create, edit or share drug libraries.

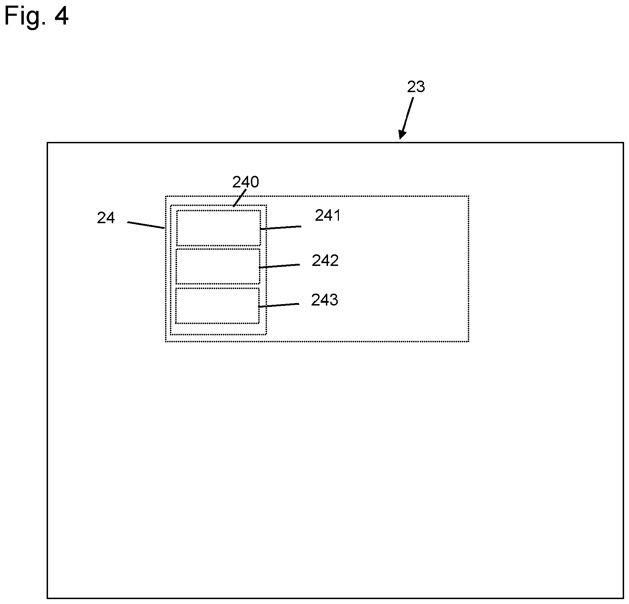

Fig. 4 illustrates schematically the setup of a user account 240 within a software application 24 installed on a computing device 23. The user account 240 is associated to a username and to a password, the combination of the user name of the password forming the user credentials. For accessing the software application 24, the user has to enter it username and the password, as it is known per se.

In addition, in association with a user account 240 additional information may be stored relating to privileges of the user and to additional limitations for user access. In particular, in association with the user account 240 information 241 may be stored relating to functions of the software application 24 which the user is allowed to access or execute, hence defining a certain set of privileges of the user. In addition, information 242 may be stored relating to the identification of a computing device 23 from which the user is authorized to access the software application 24. The information 242 may have for example the shape of a MAC address identifying a computing device 23 by its network address, the user being authorized to run the software application 24 only from the particular identified communicate dictation device 23. Further in addition, information 243 relating to a validity period of the license associated with the user account 240 may be stored, hence limiting the user to use the software application for a predefined period of time only, for example one year.

The additional information 241, 242, 243 may also be comprised in the registration code 6 and hence may be transferred to the user from the software supplier. The information 241, 242, 243 is stored in association with the user account 240 when creating the user account 240 by means of the registration process as described above.

The invention is not limited to the embodiments described above, but may be implemented in an entirely different fashion.

In particular, by means of the proposed concept a registration of a user in a software application becomes possible without having to involve a privileged user (administrator), hence making a simple workflow for registering a user in a software application possible. The registration process can be executed by an offline software application, that is without having access to a public communication network. Because the registration code can be provided in an encrypted manner, the process can be rendered safe against third-party interception, allowing for an improved licensing control.

### List of Reference Numerals

- 1: System
- 2: Hospital environment
- 20: Medical device (infusion device)
- 21: Organization device (rack)
- 22: Server
- 23: Computing (client) device
- 24: Software application
- 240: User account
- 241: Information relating to authorized functions
- 242: Identification information of a computing device
- 243: Validity information
- 3: Communication device
- 4: Communication network (internet)
- 5: External communication entity
- 6: Registration code
- 60: License key
- 61, 62: User credentials
- S1-S6: Steps
- U: User

## Claims

1. Method for registering a user (U) in a medical software application (24) used in a local communication network of a hospital environment, the method comprising:
- providing a registration code (6) to a user (U) from an external communication entity (5) via a public communication network (4), the registration code (6) containing information (60) relating to a license key and information (61, 62) relating to at least one user credential associated with the user (U),
- entering, by the user (U), the registration code (6) and at least one user credential associated with the user (U) into the software application (24) used in the local communication network of the hospital environment,
- processing, by the software application (24), the registration code (6) and the at least one user credential entered by the user (U), and
- creating a user account (240) for the user (U) in the software application (24) based on the information (61, 62) relating to the at least one user credential associated with the user (U) contained in the registration code (6).

2. Method according to claim 1, wherein the provided registration code (6) is encrypted using an encryption technology, wherein the software application (24) decrypts the registration code (6) entered by the user (U).

3. Method according to claim 1 or 2, wherein the processing, by the software application (24), includes at least one of checking the validity of the registration code (6), verifying the information relating to the license key (60) contained in the registration code (6), and verifying the information (61, 62) relating to the at least one user credential contained in the registration code with respect to the at least one user credential entered by the user (U).

4. Method according to claim 3, wherein the verifying of the information (60) relating to the license key contained in the registration code (6) includes verifying the information (60) relating to the license key with respect to a valid license key stored in the software application (24).

5. Method according to one of the preceding claims, wherein the user account (240) is created in the software application (24) if and only if the processing of the registration code (6) is successful.

6. Method according to one of the preceding claims, wherein the creating of the user account (240) in the software application (24) includes storing the information relating to the at least one user credential associated with the user (U) contained in the registration code (6).

7. Method according to one of the preceding claims, wherein the information relating to the at least one user credential associated with the user (U) contained in the registration code (6) includes at least one of a user name and a password associated with the user (U).

8. Method according to one of the preceding claims, wherein the registration code (6) contains further information relating to at least one of identification information (242) of a computing device (22, 23) on which the user (U) is authorized to execute the software application (24), information (241) related to functions of the software application (24) the user (U) is authorized to access or execute, and information (243) relating to a validity period of the license key.

9. Computing device (23) configured to execute the method according to one of claims 1 to 8.

10. Software program product implementing the method according to one of claims 1 to 8.

## Patentansprüche

1. Verfahren zum Registrieren eines Benutzers (U) in einer medizinischen Softwareanwendung (24), die in einem lokalen Kommunikationsnetzwerk einer Krankenhausumgebung verwendet wird, wobei das Verfahren Folgendes umfasst:
- Bereitstellen eines Registrierungscodes (6) von einer externen Kommunikationseinheit (5) über ein öffentliches Kommunikationsnetzwerk (4) an einen Benutzer (U), wobei der Registrierungscode (6) Informationen (60) über einen Lizenzschlüssel und Informationen (61, 62) über mindestens einen dem Benutzer (U) zugeordneten Benutzerberechtigungsnachweis enthält,
- Eingeben des Registrierungscodes (6) und mindestens eines dem Benutzer (U) zugeordneten Benutzerberechtigungsnachweises durch den Benutzer (U) in die Softwareanwendung (24), die in dem lokalen Kommunikationsnetzwerk der Krankenhausumgebung verwendet wird,
- Verarbeiten des Registrierungscodes (6) und des mindestens einen vom Benutzer (U) eingegebenen Benutzerberechtigungsnachweises durch die Softwareanwendung (24) und
- Erstellen eines Benutzerkontos (240) für den Benutzer (U) in der Softwareanwendung (24) basierend auf den im Registrierungscode (6) enthaltenen Informationen (61, 62) über den mindestens einen dem Benutzer (U) zugeordneten Benutzerberechtigungsnachweis.

2. Verfahren gemäß Anspruch 1, wobei der bereitgestellte Registrierungscode (6) unter Verwendung einer Verschlüsselungstechnologie verschlüsselt wird, wobei die Softwareanwendung (24) den vom Benutzer (U) eingegebenen Registrierungscode (6) entschlüsselt.

3. Verfahren gemäß Anspruch 1 oder 2, wobei die Verarbeitung durch die Softwareanwendung (24) mindestens eines von Folgendem beinhaltet: Überprüfen der Gültigkeit des Registrierungscodes (6), Verifizieren der Informationen über den im Registrierungscode (6) enthaltenen Lizenzschlüssel (60) und Verifizieren der Informationen (61, 62) über den mindestens einen im Registrierungscode enthaltenen Benutzerberechtigungsnachweis in Bezug auf den mindestens einen vom Benutzer (U) eingegebenen Benutzerberechtigungsnachweis.

4. Verfahren gemäß Anspruch 3, wobei das Verifizieren der im Registrierungscode (6) enthaltenen Informationen (60) über den Lizenzschlüssel das Verifizieren der Informationen (60) über den Lizenzschlüssel in Bezug auf einen in der Softwareanwendung (24) gespeicherten gültigen Lizenzschlüssel beinhaltet.

5. Verfahren gemäß einem der vorhergehenden Ansprüche, wobei das Benutzerkonto (240) genau dann in der Softwareanwendung (24) erstellt wird, wenn die Verarbeitung des Registrierungscodes (6) erfolgreich ist.

6. Verfahren gemäß einem der vorhergehenden Ansprüche, wobei das Erstellen des Benutzerkontos (240) in der Softwareanwendung (24) das Speichern der im Registrierungscode (6) enthaltenen Informationen über den mindestens einen dem Benutzer (U) zugeordneten Benutzerberechtigungsnachweis beinhaltet.

7. Verfahren gemäß einem der vorhergehenden Ansprüche, wobei die im Registrierungscode (6) enthaltenen Informationen über den mindestens einen dem Benutzer (U) zugeordneten Benutzerberechtigungsnachweis mindestens einen Benutzernamen und/oder ein dem Benutzer (U) zugeordnetes Passwort beinhalten.

8. Verfahren gemäß einem der vorhergehenden Ansprüche, wobei der Registrierungscode (6) weitere Informationen über mindestens eines von Folgendem enthält: Identifikationsinformationen (242) einer Computervorrichtung (22, 23), auf der der Benutzer (U) zur Ausführung der Softwareanwendung (24) berechtigt ist, Informationen (241) über Funktionen der Softwareanwendung (24), auf die zuzugreifen oder die auszuführen der Benutzer (U) berechtigt ist, sowie Informationen (243) über eine Gültigkeitsdauer des Lizenzschlüssels.

9. Computervorrichtung (23), die zum Implementieren des Verfahrens gemäß einem der Ansprüche 1 bis 8 konfiguriert ist.

10. Softwareprogrammprodukt, welches das Verfahren gemäß einem der Ansprüche 1 bis 8 implementiert.

## Revendications

1. Procédé d'enregistrement d'un utilisateur (U) dans une application logicielle médicale (24) utilisée dans un réseau de communication local d'un environnement hospitalier, le procédé comprenant :
- la fourniture d'un code d'enregistrement (6) à un utilisateur (U) depuis une entité de communication externe (5) par l'intermédiaire d'un réseau de communication public (4), le code d'enregistrement (6) contenant des informations (60) relatives à une clé de licence et des informations (61, 62) relatives à au moins un justificatif d'identité d'utilisateur associé à l'utilisateur (U),
- l'entrée, par l'utilisateur (U), du code d'enregistrement (6) et d'au moins un justificatif d'identité d'utilisateur associé à l'utilisateur (U) dans l'application logicielle (24) utilisée dans le réseau de communication local de l'environnement hospitalier,
- le traitement, par l'application logicielle (24), du code d'enregistrement (6) et du au moins un justificatif d'identité d'utilisateur saisi par l'utilisateur (U), et
- la création d'un compte utilisateur (240) pour l'utilisateur (U) dans l'application logicielle (24) en fonction des informations (61, 62) relatives à l'au moins un justificatif d'identité d'utilisateur associé à l'utilisateur (U) contenues dans le code d'enregistrement (6).

2. Procédé selon la revendication 1, dans lequel le code d'enregistrement (6) fourni est crypté à l'aide d'une technologie de cryptage, dans lequel l'application logicielle (24) décrypte le code d'enregistrement (6) entré par l'utilisateur (U).

3. Procédé selon la revendication 1 ou 2, dans lequel le traitement, par l'application logicielle (24), comporte au moins une des étapes consistant à vérifier la validité du code d'enregistrement (6), vérifier les informations relatives à la clé de licence (60) contenues dans le code d'enregistrement (6), et vérifier les informations (61, 62) relatives à l'au moins un justificatif d'identité d'utilisateur contenues dans le code d'enregistrement par rapport à l'au moins un justificatif d'identité d'utilisateur entré par l'utilisateur (U).

4. Procédé selon la revendication 3, dans lequel la vérification des informations (60) relatives à la clé de licence contenues dans le code d'enregistrement (6) comporte la vérification des informations (60) relatives à la clé de licence par rapport à une clé de licence valide stockée dans l'application logicielle (24).

5. Procédé selon l'une des revendications précédentes, dans lequel le compte utilisateur (240) est créé dans l'application logicielle (24) si et seulement si le traitement du code d'enregistrement (6) réussit.

6. Procédé selon l'une des revendications précédentes, dans lequel la création du compte utilisateur (240) dans l'application logicielle (24) comporte le stockage des informations relatives à l'au moins un justificatif d'identité d'utilisateur associé à l'utilisateur (U) contenues dans le code d'enregistrement (6).

7. Procédé selon l'une des revendications précédentes, dans lequel les informations relatives à l'au moins un justificatif d'identité d'utilisateur associé à l'utilisateur (U) contenues dans le code d'enregistrement (6) comportent au moins l'un d'un nom d'utilisateur et d'un mot de passe associé à l'utilisateur (U).

8. Procédé selon l'une des revendications précédentes, dans lequel le code d'enregistrement (6) contient des informations supplémentaires relatives à au moins l'une d'informations d'identification (242) d'un dispositif informatique (22, 23) sur lequel l'utilisateur (U) est autorisé à exécuter l'application logicielle (24), d'informations (241) relatives aux fonctions de l'application logicielle (24) auxquelles l'utilisateur (U) est autorisé à accéder ou qu'il est autorisé à exécuter, et d'informations (243) relatives à une période de validité de la clé de licence.

9. Dispositif informatique (23) configuré pour exécuter le procédé selon l'une des revendications 1 à 8.

10. Produit programme logiciel mettant en oeuvre le procédé selon l'une des revendications 1 à 8.
